# EUROPEAN PATENT APPLICATION

(11) **EP 4 806 362 A1**
(43) Date of publication of application: **16.09.2026**
(21) Application number: 25163445.7
(22) Date of filing: 13.03.2025
(51) Int. Cl.: A61B 6/08, A61B 6/46, A61B 6/00, A61B 6/58

(54) **AN X-RAY SYSTEM AND A METHOD OF CONFIGURING AN X-RAY SYSTEM**

(71) Applicant: Koninklijke Philips N.V., 5656 AE Eindhoven (NL)
(72) Inventor: KROENKE-HILLE, Sven, Eindhoven (NL); MENSER, Bernd, Eindhoven (NL); BYSTROV, Daniel, 5656AG Eindhoven (NL); VON BERG, Jens, Eindhoven (NL); SENEGAS, Julien Thomas, Eindhoven (NL); TIMMERS, Iris Jacoba, Eindhoven (NL)
(74) Representative: Philips Intellectual Property & Standards

(57) **Abstract**

An X-ray system has a camera for capturing an image including the field of exposure of an X-ray head of the system, and a display for displaying the image captured by the camera. A user-selected location within the displayed image is received by the system, corresponding to a desired point of the structure to be imaged. The location of the desired point of the structure to be imaged is tracked while X-ray system adjustments and/or movements of the structure to be imaged are made that change the direction of the central beam of the X-ray head relative to the structure to be imaged. A motorized driver is used to move the X-ray head (by translation or rotation) such that the central beam of the X-ray head is directed to the (tracked) desired point of the structure to be imaged.

## Description

### FIELD OF THE INVENTION

This invention relates to X-ray systems, and in particular to X-ray systems with motorized control of the position of the X-ray head.

### BACKGROUND OF THE INVENTION

Recent X-ray systems are equipped with a 3D camera and a touchscreen mounted at the X-ray head. The touchscreen enables a visual image to be viewed representing the field of irradiation of the X-ray head, so that the X-ray head position can be adjusted so that the desired region of the subject to be irradiated is in view.

There are various adjustments that can be made before the X-ray imaging takes place, such as changing the angle at which the irradiation beam enters the patient, the location at which the irradiation beam enters the patient, the patient position on a patient couch etc.

Some of these adjustments are for example made by changing the position or angular orientation of the X-ray head. These adjustments may result in long exam preparation times, in particular if the X-ray head position needs to be adapted multiple times during patient preparation. For example, if the radiographer decides to change the X-ray head angle, the central beam will penetrate the anatomy at a different location, which needs to be corrected.

An alternative to changing the position or angular orientation of the X-ray head is to move a floating patient support table so that a static X-ray head may be employed. This may be mechanically easier but is not always desirable. For example, the patient may only rest their hand on the table on a free detector. In this case, the radiographer will prefer to keep the table position fixed and move the X-ray head.

It is known to provide motorized movement of the X-ray head. For example, EP2564787 discloses the adjustment of a field of view for exposure of an X-ray system in which an image of a patient is captured on an examining table. The displayed image is presented on a touchscreen display so that a region of interest or a point of interest can be selected by a radiographer. This determines a target position of the X-ray source. The X-ray source is then driven by a motorized system to the target position.

If any system adjustments are made that change the alignment between the X-ray head and the patient, the radiographer will need to re-identify the desired region or point of interest. This creates a complicated workflow with multiple iterations.

### SUMMARY OF THE INVENTION

The invention is defined by the claims.

According to examples in accordance with an aspect of the invention, there is provided an X-ray system, comprising:
an X-ray head;
a mounting system for mounting the X-ray head to a support, the mounting system comprising a motorized driver for implementing first system adjustments comprising driving the X-ray head to different positions and/or angular orientations relative to the support;
a camera for capturing an image including the field of exposure of the X-ray head;
a display for displaying the image captured by the camera;
an X-ray detector, wherein the X-ray head and the X-ray detector are for mounting on opposite sides of a structure to be imaged; and
a controller,
wherein the controller is configured to:
   receive a user-selected location within the displayed image corresponding to a desired point of the structure to be imaged;
   track the location of the desired point of the structure to be imaged while second system adjustments are performed comprising X-ray system adjustments and/or movements of the structure to be imaged that change the direction of the central beam of the X-ray head relative to the structure to be imaged; and
   control the motorized driver to implement the first system adjustments such that the central beam of the X-ray head is directed to the desired point of the structure to be imaged.

This X-ray system performs driven movement (by which is meant translational movement and/or angular movement) of the X-ray head such that a central beam of the X-ray head is directed to a user-selected point of the structure to be imaged. The user selects this point by identifying a location of a displayed image caught by a camera.

The selected point is tracked, so that the desired X-ray head position and/or angle can be determined automatically after other system adjustments have been made. The desired central beam alignment is effectively initially locked, and after this locking the user can perform any other system adjustments. The required adjustment to the central beam alignment can be performed continuously (so that the system is always ready for an X-ray exposure), or else the user can request the control of the motorized driver at a later point in time (when the user is ready to instruct the X-ray exposure).

The move-to-position functionality enables the X-ray head movement to be controlled at the touchscreen display, and this means the display itself can be installed at positions closer to the patient and in better orientations than the usual position at the X-ray head.

The step of moving (i.e., translating or rotating) the head as first system adjustments, can be performed multiple times for a given, locked target point. This depends on the workflow. The target point can be set at one time and the motorized drive can be realized at any later time by tracking the selected point.

The controller is for example configured to receive a user instruction to implement the first system adjustments (instead of performing a continuous adjustment process). Thus, the amount of movement of the X-ray head is limited, by moving to the position and/or angle for the imaging procedure in response to a user command.

The system may further comprise a memory for storing an identification of the point of the structure to be imaged. The storage of the identified point in memory is used to enable the tracking to be performed. The locking of the central beam alignment is stored in the memory after the image acquisition. The stored information is for example also used when repositioning a patient for a retake of an X-ray image. In this way, the user does not need to adjust the central beam again if the alignment of the previous shot was already satisfactory.

The camera for example comprises a depth camera. This enables 3D positioning between the X-ray head and the desired location of the structure to be imaged. It also enables imaging of the surface topology of structure to be imaged. It also enables the positions of other objects to be taken into account for collision avoidance.

The controller may be configured to track the point of the structure by:
optical flow estimation; or
registration of point-clouds in sequential images.

Thus, any suitable image-based tracking approach may be used to identify relative movement between the structure to be imaged and the X-ray head, after the initial identification of the point of interest.

Instead of image-based tracking, other tracking approaches may be used. For example, if the X-ray system is equipped with sensors for all relevant degrees of freedom, the current system geometry will be known in terms of these system parameters. A desired point may then be tracked by calculation (i.e. by analyzing the change of these system parameters). For a static structure to be imaged, this sensor-based tracking is able to control the system parameters to track the point of interest.

A more robust tracking approach may involve a combination of image-based tracking and sensor-based tracking.

The second system adjustments (that result in misalignment) may comprise one or more selected from the list:
X-ray head tilt adjustments;
swing angle adjustments; and
position adjustments of a patient support table.

The support for example comprises a ceiling mounting and the display for example comprises a touchscreen located at the X-ray head.

The controller is for example configured to control the display to provide an image overlay at the user-selected location. The image overlay for example comprises cross hairs. It enables the user to verify that the correct point of interest has been identified.

The controller may be further configured to:
receive a user-selected desired angle of incidence to the desired point of the structure to be imaged;
track the angle of incidence while the second system adjustments is performed; and
control the X-ray system such that central beam of the X-ray head is directed to the desired point of the structure to be imaged with the desired angle of incidence.

In this way, the controller may ensure that the point of interest is aligned with the central beam of the X-ray head, and that a desired angle of incidence is achieved. The control of the angle of incidence may be achieved by the first system adjustments (for example it may include tilt control), or it may be a separate process implemented by the controller, wherein the separate process and the first system adjustments operate together to achieve the imaging of the point of interest at the desired angle of incidence.

The controller is for example further configured to control the X-ray head to perform X-ray imaging after moving the X-ray head.

The system for example comprises a patient support and the X-ray detector is fixed to the patient support. However, the X-ray detector may instead be a movable ("free") detector, and the controller may be configured to locate the movable X-ray detector in the camera image and to limit the positions and/or angular orientations to which the X-ray head is driven during the first system adjustments such that the X-ray detector remains aligned with the field of exposure of the X-ray head. In this case, the system can ensure that driving of the X-ray head does not result in the detector being positioned outside the field of exposure of the X-ray head. A warning may additionally be provided when the movement of the X-ray detector has been limited in this way. The automatic localization of the movable X-ray detector can be realized e.g. by classical computer vision algorithms and optical marker (e.g. QR codes) attached to the detector cover, or by machine learning approaches e.g. detection of the detector corner points by stacked hour-glass convolutional neural networks.

The invention also provides a method of configuring an X-ray system, the X-ray system comprising:
an X-ray head;
a mounting system for mounting the X-ray head to a support, the mounting system comprising a motorized driver for implementing first system adjustments comprising driving the X-ray head to different positions and/or angular orientations relative to the support;
a camera for capturing an image including the field of exposure of the X-ray head;
a display for displaying the image captured by the camera; and
an X-ray detector, wherein the X-ray head and the X-ray detector are for mounting on opposite sides of a structure to be imaged,
wherein the method comprises:
   receiving a user-selected location within the displayed image corresponding to a desired point of the structure to be imaged;
   tracking the location of the desired point of the structure to be imaged while second system adjustments are performed comprising X-ray system adjustments and/or movements of the structure to be imaged that change the direction of the central beam of the X-ray head relative to the structure to be imaged; and
   controlling the motorized driver to implement the first system adjustments such that the central beam of the X-ray head is directed to the desired point of the structure to be imaged.

The method may comprise receiving a user instruction to implement the first system adjustments. Thus, the driving of the X-ray head may be delayed until all X-ray system adjustments have been made.

The method for example comprises storing an identification of the desired point of the structure to be imaged.

The invention also provides a computer program comprising computer program code means which is adapted, when said program is run on a controller of an X-ray system, to perform the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an X-ray system; and
Fig. 2 shows a method of configuring and using an X-ray system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an X-ray system having a camera for capturing an image including the field of exposure of an X-ray head of the system, and a display for displaying the image captured by the camera. A user-selected location within the displayed image is received by the system, corresponding to a desired point of the structure to be imaged. The location of the desired point of the structure to be imaged is tracked while X-ray system adjustments and/or movements of the structure to be imaged are made that change the direction of the central beam of the X-ray head relative to the structure to be imaged. A motorized driver is used to move the X-ray head (by translation or rotation) such that the central beam of the X-ray head is directed to the (tracked) desired point of the structure to be imaged.

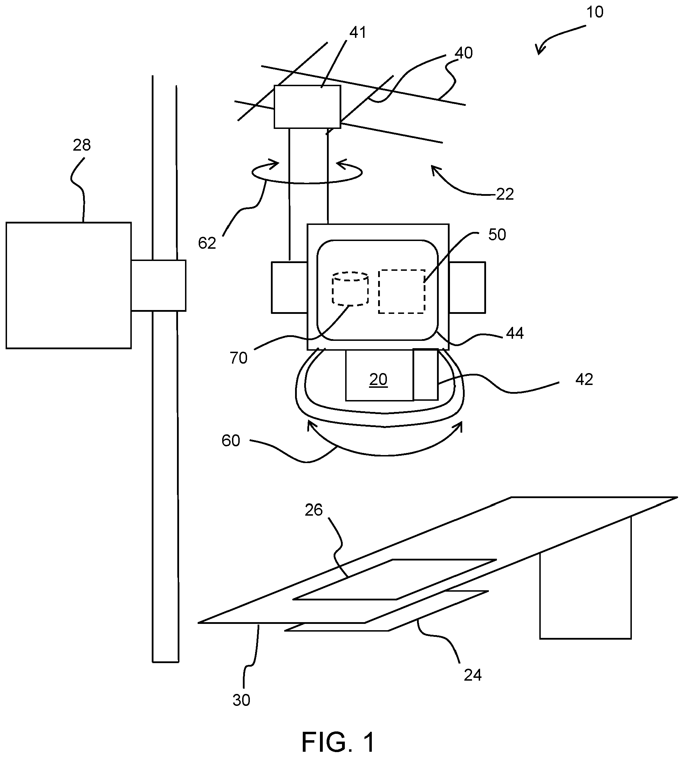

Fig. 1 shows an X-ray system 10. It comprises an X-ray head 20 mounted to a mounting system 22.

The X-ray 20 head faces a detector, such that the X-ray head and the X-ray detector on opposite sides of a structure to be imaged, typically a part of the body of a patient. Fig. 1 shows three examples of possible detector. A first example is a detector 24 that is fixed to a patient couch 30. The detector 24 is for imaging a particular region of the body, such as the chest, and it is positioned relative to the patient couch to be aligned with that portion of the patient when the patient lies on the patient couch. A second example is a so-called free detector 26 which can be moved freely, for example placed on the patient couch. The subject for example places a limb on the free detector 26 for imaging that limb. Free detectors are often used for musculoskeletal exams. The third example is a detector 28 for a standing patient, for example for an upright chest X-ray.

The mounting system 22 enables movement of the X-ray head. This movement may comprise translational movement, for example in a plane parallel to a plane of the detector, or it may comprise angular adjustment so that the angle of incidence of the X-ray beam on the patient is adjusted. It may comprise a combination of translational and angular adjustment. In this description, the term "movement" is intended to cover all of these possibilities.

Fig. 1 schematically shows one example in which the mounting system comprises orthogonal rails 40 to enable in-plane translational movement, and in the example shown, the mounting system is fixed to a support in the form of a ceiling.

However, the mounting system may connect to other supports, such as a floor standing support for the standing patient detector 28 or a wall mounted support. Thus, in general, the mounting system 22 is for mounting the X-ray head 20 to any suitable support.

The mounting system comprises a motorized driver 41 for driving the X-ray head to different positions and/or angular orientations relative to the support, i.e. along the rails 40 in the example shown.

In general, the motorized driver 41 of the mounting system 22 is for implementing first system adjustments. The first system adjustments comprise driving the X-ray head to different positions and/or angular orientations relative to the support. In the example outlined above, the first system adjustments involve driving longitudinal and transverse movement of the X-ray head relative to the patient couch, i.e. movements in a plane parallel to the patient couch, in particular in a horizontal plane. However, as mentioned above, the first system adjustments may involve angular adjustments of the X-ray head, or a combination of translational movement and angular adjustment such as a transverse movement and a tilt angle control.

A camera 42 is used to capture an image including the field of exposure of the X-ray head 40. The camera 42 is preferably a 3D (depth) camera. The camera image enables the user (radiographer) to visually inspect the region of the patient that will be imaged.

A display 44 is used to display the image captured by the camera 42. The display 44 is for example a touchscreen display, so that the user can identify a point of interest on the display screen. However, a mouse or other pointer could instead be used for this purpose. The selected point of interest is for example identified to the user by an image overlay at the user-selected location, such as cross hairs.

A controller 50 is used to control the X-ray head 20 and process the detected image, as well as process the touchscreen input, generate the display output etc.

In operation, a patient is positioned in front of the X-ray head 20, e.g. on the patient couch 30 or in front of the standing patient detector 28. The user selects from the displayed image the point (or region) of interest, namely the region that is to be in the center of the X-ray image. The controller 50 receives this user-selected location within the displayed image corresponding to the desired point of the structure to be imaged.

The location of the desired point of the structure to be imaged is, from that time, tracked, while X-ray system adjustments and/or movements of the patient are made. These are second system adjustments. These second adjustments are for example to parameters other than those controlled as part of the first system adjustments. They are all adjustments that change the direction of the central beam of the X-ray head 20 relative to the structure to be imaged. Thus, the point at which the central beam will penetrate the patient is shifted (and in some cases also the angle of incidence). In other words, these second system adjustments disturb the X-ray head alignment. They may be manual adjustments to the X-ray system or to the patient position or they may be automated adjustments of the X-ray system. By tracking the point of interest, no further user input to the touchscreen is needed to allow the motorized driver to move the X-ray head such that the central beam of the X-ray head is directed to the desired point of the structure to be imaged.

The movements made as part of the first system adjustments may be considered to comprise first degrees of freedom according to which the system may be configured, and the adjustments that constitute the second system adjustments may be considered to comprise second degrees of freedom according to which the system may be configured. The first and second degrees of freedom are preferably different, i.e. mutually exclusive. Thus, the first system adjustments involve changing a first set of system parameters and they are implemented by the motorized driver. The second system adjustments involve changing a second set of system parameters (preferably different to the system parameters of the first set). The second set of system parameters may be manual adjustments, but equally they could be made by an automated system separate to the motorized driver.

There may, however, be overlap between the first and second system adjustments. For example, the radiographer may manually move the X-ray head to give better access to the anatomy (as one of the second system adjustments), and the first system adjustments may also involve movement of the X-ray head back to the correct position and/or orientation.

By way of example, when the first system adjustments involve controlling translational movement as in the example above, the second system adjustments may involve motorized tilt control. Furthermore, the motorized tilt control may itself be part of an automated tilt control approach, for maintaining a desired angle of incidence of the central X-ray beam to the patient.

The system parameters that may be divided in different ways into the first and second sets of system parameters for example comprise translational movement of the X-ray head along three orthogonal axes, and X-ray head angle adjustments, such as swing and tilt rotations (as discussed below), and positioning of the structure to be imaged.

The driven movement (translational or rotational) is achieved by having a known system geometry and by having a known calibrated position of the camera in relation to the system geometry. The x,y pixel coordinates of the requested position on the display can in this way be translated to a 3D point in the coordinate system of the support (e.g. mounted to a ceiling). From the differences between the known current X-ray head position (in 3D) and this 3D point, translational movement instructions and/or angular movement instructions can be derived and realized.

In one example, the first system adjustments comprise driven movement of the X-ray head in a plane parallel to a plane of the detector. This gives two motion axes. The angle of the beam incidence on the detector is thus not changed by these driven X-ray head movements, and a tilt of the X-ray head and the X-ray head movement are thereby decoupled. The tilt angle is a known system parameter, and it is taken into account when translating the user-selected screen location to the X-ray head movement. The driven movement preferably additionally includes the third orthogonal axis, namely perpendicularly towards or away from the detector, so changing the distance from the X-ray head to the patient.

Fig. 1 shows the tilt as arrow 60. In the case of a patient couch, it is a rotation about an axis perpendicular to the length direction of the patient couch, so moves the field of view along the couch length and changes the angle of incidence to the patient couch. A swing is shown as arrow 62. In the case of a patient couch, it is a rotation about an axis perpendicular to the plane of the patient couch, so moves the field of view to different positions over the patient couch without changing the angle of incidence to the patient couch.

By tracking the user-selected point, the desired X-ray head position can be determined after these system adjustments (tilt, swing, movement of the patient, movement of the patient couch) have been made.

As explained above, the second system adjustments may be considered to be additional degrees of freedom in the control of the system (i.e. additional to translational movement and/or angular control of the first system adjustments). The desired central beam alignment is effectively initially locked, and after this locking the user can perform any other system adjustments.

In one example, once the user has selected a point of interest, the X-ray head may be moved by automated motorized control to align the central beam with the point of interest. This enables the user to verify from the updated camera image that they have correctly selected the desired point of interest. After the additional (second) system adjustments, the user can then request realignment, and automatic motorized X-ray head movement is performed.

In another example, once the user has selected a point of interest, the identified point of interest is simply stored in a memory 70. After the additional (second) system adjustments, the user instructs X-ray head control and the stored information is used for the automatic motorized X-ray head movement (i.e. the first system adjustments).

In another example, once the user has selected a point of interest, the X-ray head may be controlled to align the central beam with the point of interest, and it is then controlled continuously to maintain real-time alignment with the point of interest while the second system adjustments are being made.

When the motorized movement is performed at the instruction of the user (rather than continuously) it can nevertheless be performed multiple times for a given, locked point of interest. This depends on the workflow. The target point can be set at one time and the motorized drive can be realized at any later time by tracking the selected point.

By storing an identification of the point of interest, the motorized drive of the X-ray head may be repeated between multiple imaging operations, with patient movement (e.g. on and off the patient couch) between those imaging operations. This enables the stored information to be used for a retake of an X-ray image after patient repositioning.

It is mentioned above that the X-ray detector may be a movable ("free") detector. The controller may be configured to locate the movable X-ray detector in the camera image. Using state-of-the-art machine-learning or computer vision algorithms, the free detector can be localized in the camera image (for example using bounding box detection, corner-point detection, segmentation, regression of center position...) as long as it is visible or partially visible in the field-of-view of the camera.

When the motorized control of the X-ray head takes place (the first system adjustments described above), a verification can then made that the X-ray tube head position and angular orientation still result in irradiation of the detector. The adjustments made to the position or angle during the first system adjustments can for example be limited in such a way that the detector remains irradiated. A warning may be provided if the detector is in a position that means that imaging of a desired point of interest, at a desired angle of entry, is not possible.

The use of a 3D camera enables the system to further check for potential collision with devices or persons during the motorized movement of the X-ray head, by comparing the position in 3D of devices and persons and the planned trajectory of the X-ray head to reach its desired position. Pre-stored information about the 3D shape and volume of the mechanical parts attached to the X-ray head and that will move together with the X-ray head can further be incorporated for this collision check.

Alternatively, the 3D camera data can be used to compute the trajectory that would avoid collisions with detected devices and persons, by adapting accordingly the sequence of translations in the three spatial dimensions. The collision check is for example updated continuously as the X-ray head is moved to its desired position, using real-time 3D data.

Other safety mechanisms like proximity sensors (radar, lidar etc.) or pressure sensors can further be used to either prevent collision or minimize the danger, by stopping the movement of the X-ray head as soon as a contact is detected.

Computer-vision algorithms (potentially also including machine learning components) can also be used to trace the desired X-ray head position in the camera image upon X-ray head movement. Thereby, the movement can be controlled in such a way that the desired position is reached up to a given tolerance. This can be realized by using only an RGB camera (e.g. by optical flow estimation) or by a camera measuring also the depth. In the latter case, point cloud registration techniques (e.g. iterative closest point, ICP, based registration) can be used for tracing the desired position in each frame (given sufficient 3D surface structure, e.g. curvature).

In general, the tracking of the point of interest may be performed by any suitable known image processing approach. As mentioned above, one general example is to use optical flow estimation. Another is to use registration of point-clouds in sequential images.

The X-ray head may be movable manually as well as under the automated motorized control.

In the example above, the first system adjustments involve providing movements of the X-ray head such that a same point of the anatomy of the patient remains aligned with the central beam of the X-ray head. The first system adjustments may additionally maintain a same angle of incidence of the X-ray beam. Thus, when a user-selected location is provided, the location as well as the angle of incidence is effectively locked and then tracked.

The angle of incidence refers to the angle between the central beam and the local surface of the anatomy to be imaged. The user may for example set the desired angle of incidence by tilting the X-ray tube or by (re)positioning the patient (for example using pillows etc. to locally lift the anatomy). A measurement of the current angle of incidence is then triggered by the user interface of the system, for example as part of the same user instruction to lock the location of the central beam. Thus, there may be a single instruction to lock both the identified screen location and the current angle of incidence, or there may be separate sequential instructions for identifying the location of the point of interest and for locking the angle of incidence.

To measure the selected angle of incidence, the local surface topography can be obtained by sufficiently precise depth sensing. Thus, in one example, the angle of incidence is measured by using analysis of the images captured by the depth camera at the time when the user triggers the locking function. Using the depth data in the vicinity of the central beam, the local surface topography can be interpolated and, using a spatial calibration of the depth camera with the geometry of the X-ray head, the current angle of incidence can be calculated.

In another example, the angle of incidence is measured with respect to the detector using either measurable system parameters (such as the current geometrical relationship between the X-ray head and the detector) or, in the case of a movable X-ray detector, by localizing the latter in the camera image and using a spatially calibrated depth-camera signal to estimate the orientation of the movable X-ray detector plane relative to the central beam.

The location of the point of interest and the angle of incidence are stored in memory so that they may both be tracked.

After system adjustments have been made (the second system adjustments), the motorized driving of the X-ray head ensures that the desired angle of incidence is achieved and the point of interest is imaged.

In one example, the angle of incidence may be controlled by a separate automated control process. Thus, after the second system adjustments have been made, the motorized control of the X-ray head may provide translational position control of the X-ray head and the separate automated control process may provide tilt control. In combination, the two automated control processes ensure that the central beam of the X-ray aligns with the point of interest of the patient's anatomy and has a desired angle of incidence.

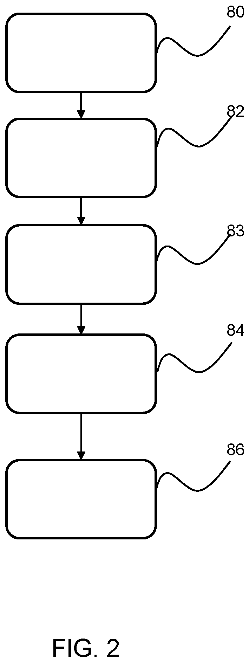

Fig. 2 shows a method of configuring the X-ray system as described above.

The method comprises:
in step 80, receiving a user-selected location with a displayed image corresponding to a desired point of the structure to be imaged;
in step 82, tracking the location of the desired point of the structure to be imaged while the second system adjustments are performed comprising X-ray system adjustments and/or movements of the structure to be imaged that change the direction of the central beam of the X-ray head relative to the structure to be imaged; and
in step 84, controlling the motorized driver to implement the first system adjustments such that the central beam of the X-ray head is directed to the desired point of the structure to be imaged.

The X-ray imaging takes place in step 86.

As mentioned above, the adjustments that are made as part of the second system adjustments may be manual or automatic or a combination of manual and automatic adjustments.

One example of a possible use case is set out below.

As a first step, a radiographer places a patient for knee anterior-posterior exam on a patient table, with a movable (free) detector beneath the knee.

The radiographer then locks the desired central beam location on the knee by viewing the displayed image and indicating the desired central beam alignment using the touchscreen function of the display.

By way of example, the radiographer may then realize that a pillow under the lower leg is needed for realizing the optimal angle of incidence and the radiographer places the pillow. This is a second system adjustment, and it is a manual adjustment in this example that moves the location of the structure to be imaged. This will of course result in a misalignment of the central beam.

The radiographer then triggers the motorized X-ray head control to provide the previously locked central beam alignment, This involves automated movement of the X-ray head, and this constitutes the first system adjustments.

In this example, the method thus comprises receiving a user instruction in step 83 to implement the first system adjustments. Thus, the amount of movement (translation and/or rotation) of the X-ray head is limited to movement only once the other X-ray system adjustments have been made, such as the movement of the structure to be imaged in the example above.

It is noted that there the first system adjustments may be made (at least) twice- once before the second system adjustments are made and once after the second system adjustments are made. Thus, there may be an initial movement once the desired point of the structure has been selected followed by a final movement once the second system adjustments have been made.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An X-ray system (10), comprising:
an X-ray head (20);
a mounting system (22) for mounting the X-ray head (20) to a support, the mounting system (22) comprising a motorized driver (41) for implementing first system adjustments comprising driving the X-ray head to different positions and/or angular orientations relative to the support;
a camera (42) for capturing an image including the field of exposure of the X-ray head (20);
a display (44) for displaying the image captured by the camera (42);
an X-ray detector (24,26,28), wherein the X-ray head and the X-ray detector are for mounting on opposite sides of a structure to be imaged; and
a controller (50),
wherein the controller (50) is configured to:
receive a user-selected location within the displayed image corresponding to a desired point of the structure to be imaged;
track the location of the desired point of the structure to be imaged while second system adjustments are performed comprising X-ray system adjustments and/or movements of the structure to be imaged that change the direction of the central beam of the X-ray head relative to the structure to be imaged; and
control the motorized driver (41) to implement the first system adjustments such that the central beam of the X-ray head is directed to the desired point of the structure to be imaged.

2. The system of claim 1, wherein the controller (50) is configured to receive a user instruction to control the motorized driver to implement the first system adjustments.

3. The system of claim 1 or 2, further comprising a memory (70) for storing an identification of the point of the structure to be imaged.

4. The system of any one of claims 1 to 3, wherein the camera (42) comprises a depth camera.

5. The system of any one of claims 1 to 4, wherein the controller (50) is configured to track the point of the structure by:
optical flow estimation; or
registration of point-clouds in sequential images.

6. The system of any one of claims 1 to 5, wherein the second system adjustments comprise one or more selected from the list:
X-ray head tilt angle adjustments;
swing angle adjustments; and
position adjustments of a patient support table.

7. The system of any one of claims 1 to 6, wherein the display (44) comprises a touch screen located at the X-ray head.

8. The system of any one of claims 1 to 7, wherein the controller (50) is configured to control the display (44) to provide an image overlay at the user-selected location.

9. The system of any one of claims 1 to 8, wherein the controller (50) is further configured to:
receive a user-selected desired angle of incidence to the desired point of the structure to be imaged;
track the angle of incidence while the second system adjustments is performed; and
control the X-ray system such that central beam of the X-ray head is directed to the desired point of the structure to be imaged with the desired angle of incidence.

10. The system of any one of claims 1 to 9, wherein the controller (50) is further configured to control the X-ray head (20) to perform X-ray imaging after implementing the first system adjustments.

11. The system of any one of claims 1 to 10, wherein:
the system comprises a patient support (30) and the X-ray detector (24) is fixed to the patient support (30); or
the X-ray detector (26) is movable and the controller is configured to locate the movable X-ray detector in the camera image and to limit the positions and/or angular orientations to which the X-ray head is driven during the first system adjustments such that the X-ray detector remains aligned with the field of exposure of the X-ray head.

12. A method of configuring an X-ray system, the X-ray system comprising:
an X-ray head (20);
a mounting system (22) for mounting the X-ray head to a support, the mounting system comprising a motorized driver (41) for implementing first system adjustments comprising driving the X-ray head to different positions and/or angular orientations relative to the support;
a camera (42) for capturing an image including the field of exposure of the X-ray head;
a display (44) for displaying the image captured by the camera; and
an X-ray detector, wherein the X-ray head and the X-ray detector are for mounting on opposite sides of a structure to be imaged,
wherein the method comprises:
(80) receiving a user-selected location within the displayed image corresponding to a desired point of the structure to be imaged;
(82) tracking the location of the desired point of the structure to be imaged while second system adjustments are performed comprising X-ray system adjustments and/or movements of the structure to be imaged that change the direction of the central beam of the X-ray head relative to the structure to be imaged; and
(84) controlling the motorized driver to implement the first system adjustments such that the central beam of the X-ray head is directed to the desired point of the structure to be imaged.

13. The method of claim 12, comprising (83) receiving a user instruction to implement the first system adjustments.

14. The method of claim 12 or 13, comprising storing an identification of the desired point of the structure to be imaged.

15. A computer program comprising computer program code means which is adapted, when said program is run on a controller of an X-ray system, to perform the method of any one of claims 12 to 14.
